# EUROPEAN PATENT APPLICATION

(11) **EP 3 889 140 A1**
(43) Date of publication of application: **06.10.2021**
(21) Application number: 19890420.3
(22) Date of filing: 26.11.2019
(51) Int. Cl.: C07D 241/46

(54) **PHENAZINE-BASED COMPOUND, ELECTROLYTIC LIQUID COMPRISING SAME FOR REDOX FLOW BATTERY, AND REDOX FLOW BATTERY**

(30) Priority: 30.11.2018 KR 20180152003
(71) Applicant: Lotte Chemical Corporation, Seoul, 05551 (KR); Seoul National University R & DB Foundation, Seoul 08826 (KR)
(72) Inventor: LEE, Yong Hee, Daejeon 34110 (KR); KANG, Tae Hyuk, Daejeon 34110 (KR); SHIN, Dong Myoung, Daejeon 34110 (KR); KWON, Gi Yun, Seoul 08826 (KR); LEE, Kyu Nam, Seoul 08826 (KR); KANG, Ki Suk, Seoul 08826 (KR); PARK, Soo Young, Seoul 08826 (KR); KWON, Ji Eon, Seoul 08826 (KR)
(74) Representative: Germain Maureau
(86) International application number: PCT/KR2019/016361
(87) International publication number: WO 2020/111725

(57) **Abstract**

The present invention relates to: a phenazine-based compound having a particular substituent; an electrolytic liquid comprising same for a redox flow battery; and a redox flow battery. The phenazine-based compound is a multi-redox organic substance capable of inducing a two-stage reversible redox reaction and shows high solubility in a non-aqueous solvent. Thus, the employment of the phenazine-based compound can provide an electrolytic liquid for a redox flow battery, capable of expressing high energy density, and a redox flow battery.

## Description

### [Technical Field]

The present invention relates to a phenazine-based compound, a redox flow battery electrolyte including the same, and a redox flow battery. More particularly, the present invention relates to a phenazine-based compound which is a multi-redox material and has excellent solubility in a solvent, a redox flow battery electrolyte including the same, and a redox flow battery.

### [Background Art]

A redox flow battery (RFB) is a secondary battery that uses the reduction-oxidation (redox) reaction of an active material, that is, a redox couple, dissolved in an electrolyte. Since the capacity of the redox flow battery can be increased, and the redox flow battery has low maintenance costs, can be operated at room temperature, and capacity and output can be independently designed, it is attracting attention as a next-generation mass storage device.

An electrolyte, which is one of the core materials of a redox flow battery, is a solution obtained by dissolving an active material capable of a reversible redox reaction in a solvent, and various redox flow batteries can be configured depending on the type of active material. Major redox couples developed to date include Fe/Cr, V/V, V/Br, Zn/Br, and Zn/Ce, which are aqueous redox couples using water as a solvent. However, in the case of a redox flow battery including such an aqueous electrolyte, there is a disadvantage in that an operating potential is limited to a water decomposition potential region, and thus, energy density is low due to a low driving voltage.

In order to overcome these shortcomings, a solvent for an electrolyte has been replaced with a non-aqueous solvent, and there have been research efforts to develop an organic active material which, when dissolved in the above-described non-aqueous solvent, is capable of achieving high energy density by undergoing an oxidation-reduction reaction at a cathode and an anode.

As a conventional technique, Patent Document 1 (Korean Patent Publication No. 10-2014-0071603) discloses a redox flow battery electrolyte including an all-organic redox couple. The redox flow battery of Patent Document 1 includes a non-aqueous solvent as a solvent for an electrolyte, in which case, a high operating voltage can be obtained. Accordingly, the battery can exhibit higher energy efficiency than a flow battery including a transition metal redox couple.

However, the organic active materials disclosed in Patent Document 1 have a limitation in that they are single redox materials only capable of a single redox reaction. That is, referring to the cyclic cyclic voltammograms of electrolytes including the active materials disclosed in Patent Document 1, it can be seen that only one redox peak is produced per material. In this case, in order to achieve high energy density, it is required that an electrolyte with a high concentration prepared by dissolving a large amount of the active material is used. However, when the concentration is increased, the viscosity of the electrolyte increases, which is fatal to cell performance.

As another conventional technique, Patent Document 2 (US20170062842 A1) discloses a redox flow battery including an organic redox material such as 5,10-dimethyl-5,10-dihydrophenazine (DMPZ). The DMPZ is a multi-redox material exhibiting two-stage reversible electrochemical activity since two nitrogen moieties serve as redox motifs. That is, referring to a cyclic cyclic voltammogram obtained from an electrolyte in which the DMPZ is dissolved, it can be seen that two redox peaks can be produced from a single DMPZ material. Therefore, at the same electrolyte concentration, the DMPZ can achieve a higher energy density than the organic active materials disclosed in Patent Document 1. However, since the DMPZ has low solubility in a non-aqueous solvent such as acetonitrile, a redox flow battery including an electrolyte prepared using the DMPZ has a disadvantage in that the maximum attainable energy density is low.

### [Related-Art Documents]

### [Patent Documents]

(Patent Document 1) KR1020140071603 A
(Patent Document 2) US20170062842 A1

### [Disclosure]

### [Technical Problem]

The present invention is directed to providing a phenazine-based compound, which is a multi-redox organic material capable of causing a two-stage reversible redox reaction and has high solubility in a solvent.

In addition, the present invention is directed to providing a redox flow battery electrolyte including the phenazine-based compound; and a redox flow battery.

### [Technical Solution]

One aspect of the present invention provides a phenazine-based compound represented by the following Chemical Formula 1.

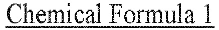

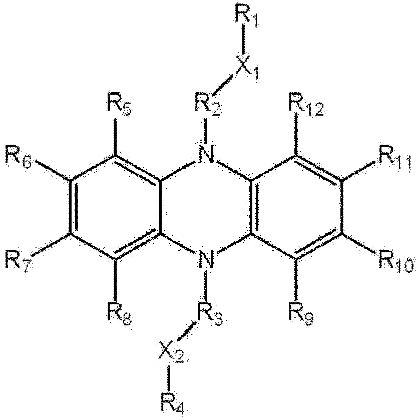

In Chemical Formula 1,
X₁ and X₂ are the same or different, and are each independently oxygen (O), sulfur (S), or silicon (Si),
R₁ and R₄ are the same or different, and are each independently an alkyl group unsubstituted or substituted with an acetal group or an ether group; an aryl group unsubstituted or substituted with an acetal group or an ether group; an alkylaryl group unsubstituted or substituted with an acetal group or an ether group; or an arylalkyl group unsubstituted or substituted with an acetal group or an ether group,
R₂ and R₃ are the same or different, and are each independently an alkylene group unsubstituted or substituted with an acetal group or an ether group, and
R₅, R₆, R₇, R₈, R₉, R₁₀, R₁₁, and R₁₂ are the same or different, and are each independently hydrogen, a halogen group, a hydroxyl group (-OH), a nitro group (-NO₂), a trifluoromethyl group (-CF₃), an alkoxy group, an alkyl group, a heteroalkyl group, a haloalkyl group, an aryl group, an alkylaryl group, or an arylalkyl group.

Another aspect of the present invention provides a redox flow battery electrolyte, which includes a solute including the above-described phenazine-based compound; and a solvent.

Still another aspect of the present invention provides a redox flow battery, which includes the above-described electrolyte.

### [Advantageous Effects]

A phenazine-based compound of the present invention is a multi-redox material capable of causing a two-stage reversible redox reaction. Therefore, an electrolyte of the present invention including the phenazine-based compound as an active material can provide a redox flow battery with a higher energy density than an electrolyte including a conventional single-redox material only capable of causing a single-stage redox reaction.

In addition, the phenazine-based compound of the present invention has remarkably high solubility in a non-aqueous solvent. Accordingly, a larger amount of the phenazine-based compound can be dissolved in a non-aqueous solvent, and an electrolyte for a redox flow battery having a high maximum energy density can be provided.

### [Description of Drawings]

FIG. 1 shows the ¹H-nuclear magnetic resonance (NMR) analysis results of 5,10-bis(2-methoxyethyl)-5,10-dihydrophenazine (BMEPZ) produced according to Example 1 of the present invention.
FIG. 2 shows the ¹³C-NMR analysis results of BMEPZ produced according to Example 1 of the present invention.
FIG. 3 is a cyclic voltammogram of a cathode electrolyte prepared according to Example 2.
FIG. 4 is a cyclic voltammogram of an anode electrolyte prepared according to Example 3.
FIG. 5 is a cyclic voltammogram of a combined electrolyte prepared according to Example 3.

### [Best Mode]

Hereinafter, the present invention will be described in detail.

In the present specification, when it is stated that a part "includes," "comprises," or "contains" a component, it means that the part can include, rather than exclude, other additional components unless specifically stated to the contrary.

In addition, the term "alkyl group" described herein refers to a monovalent linear, branched, or cyclic saturated hydrocarbon group consisting only of carbon and hydrogen atoms, and examples thereof include a methyl group, an ethyl group, a propyl group, an isopropyl group, a butyl group, an isobutyl group, a t-butyl group, a pentyl group, a hexyl group, an octyl group, a dodecyl group, a cyclopropyl group, a cyclobutyl group, a cyclopentyl group, a cyclohexyl group, a cycloheptyl group, a cyclooctyl group, a cyclononyl group, a cyclodecyl group, and the like, but the present invention is not limited thereto.

In addition, the term "aryl group" described herein refers to an organic group derived from an aromatic hydrocarbon by the removal of one hydrogen, and includes a monocyclic system or a fused ring system. Specific examples of the aryl group include a phenyl group, a naphthyl group, a biphenyl group, an anthryl group, a fluorenyl group, a phenanthryl group, a triphenylenyl group, a pyrenyl group, a perylenyl group, a chrysenyl group, a naphthacenyl group, a fluoranthenyl group, and the like, but the present invention is not limited thereto.

In addition, the term "alkylaryl group" described herein refers to an organic group in which one or more hydrogen atoms of an aryl group have been substituted with an alkyl group, and examples thereof include a methylphenyl group, an ethylphenyl group, an n-propylphenyl group, an isopropylphenyl group, an n-butylphenyl group, an isobutylphenyl group, a t-butylphenyl group, and the like, but the present invention is not limited thereto.

In addition, the term "arylalkyl group" described herein refers to an organic group in which one or more hydrogen atoms of an alkyl group have been substituted with an aryl group, and examples thereof include a phenylpropyl group, a phenylhexyl group, and the like, but the present invention is not limited thereto.

In the present specification, examples of the "alkylaryl group" and "arylalkyl group" may be the same as those exemplified above for the alkyl group and the aryl group, but the present invention is not limited thereto.

In the present specification, "acetal group" refers to an organic group formed by a bond between an alcohol and an aldehyde, that is, a substituent having two ether (-OR) bonds on one carbon, and examples thereof include a methoxymethoxy group, a 1-methoxyethoxy group, a 1-methoxypropyloxy group, a 1-methoxybutyloxy group, a 1-ethoxyethoxy group, a 1-ethoxypropyloxy group, a 1-ethoxybutyloxy group, a 1-(n-butoxy)ethoxy group, a 1-(isobutoxy)ethoxy group, a 1-(secondary butoxy)ethoxy group, a 1-(tertiary butoxy)ethoxy group, a 1-(cyclohexyloxy)ethoxy group, a 1-methoxy-1-methylmethoxy group, a 1-methoxy-1-methylethoxy group, and the like, but the present invention is not limited thereto.

In the present specification, "ether group" is an organic group having one or more ether bonds (-O-), and examples thereof include a 2-methoxyethyl group, a 2-ethoxyethyl group, a 2-butoxyethyl group, a 2-phenoxyethyl group, a 2-(2-methoxyethoxy)ethyl group, a 3-methoxypropyl group, a 3-butoxypropyl group, a 3-phenoxypropyl group, a 2-methoxy-1-methylethyl group, a 2-methoxy-2-methylethyl group, a 2-methoxyethyl group, a 2-ethoxyethyl group, a 2-butoxyethyl group, a 2-phenoxyethyl group, and the like, but the present invention is not limited thereto.

In the present specification, "alkylene group" refers to a divalent atomic group formed by excluding, from a saturated aliphatic hydrocarbon, two hydrogen atoms bonded to two different carbon atoms, and examples thereof include a methylene group, an ethylene group, a propylene group, a butylene group, and the like, but the present invention is not limited thereto.

In the present specification, "halogen group" means fluorine (F), chlorine (Cl), bromine (Br), or iodine (I).

In the present specification, "alkoxy group" refers to an atomic group formed of a linear, branched, or cyclic alkyl group and an oxygen atom bonded thereto, and examples thereof include a methoxy group, an ethoxy group, an n-propoxy group, an isopropoxy group, an isopropyloxy group, an n-butoxy group, an isobutoxy group, a t-butoxy group, and the like, but the present invention is not limited thereto.

In the present specification, "heteroalkyl group" refers to an alkyl group in which a carbon atom (including a terminal carbon atom) in the alkyl group has been substituted with one or more heteroatoms, examples of which include nitrogen (N), oxygen (O), sulfur (S), phosphorus (P), and the like, but the present invention is not limited thereto.

In the present specification, "haloalkyl group" refers to an organic group in which one or more hydrogen atoms of an alkyl group have been substituted with a halogen group, and examples thereof include a fluoromethyl group, a bromomethyl group, a chloromethyl group, a trifluoromethyl group, and the like, but the present invention is not limited thereto.

In the present specification, "multi-redox material" refers to a material which produces two or more reversible redox peaks when an electrolyte is scanned at a speed of 50 mVs⁻¹ or more using cyclic voltammetry, and "single-redox material" refers to a material that only produces one irreversible redox peak under the same conditions.

One aspect of the present invention provides a phenazine-based compound represented by the following Chemical Formula 1.

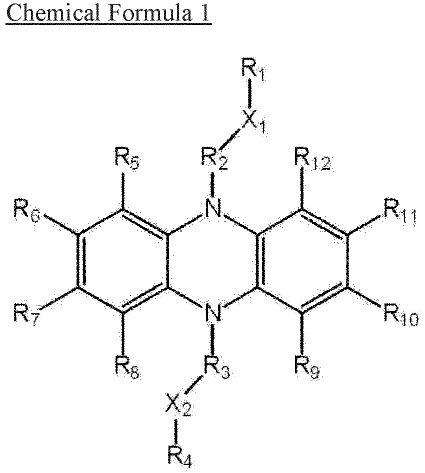

In Chemical Formula 1,
X₁ and X₂ are the same or different, and are each independently O, S, or Si,
R₁ and R₄ are the same or different, and are each independently an alkyl group unsubstituted or substituted with an acetal group or an ether group; an aryl group unsubstituted or substituted with an acetal group or an ether group; an alkylaryl group unsubstituted or substituted with an acetal group or an ether group; or an arylalkyl group unsubstituted or substituted with an acetal group or an ether group,
R₂ and R₃ are the same or different, and are each independently an alkylene group unsubstituted or substituted with an acetal group or an ether group, and
R₅, R₆, R₇, R₈, R₉, R₁₀, R₁₁, and R₁₂ are the same or different, and are each independently hydrogen, a halogen group, a hydroxyl group (-OH), a nitro group (-NO₂), a trifluoromethyl group (-CF₃), an alkoxy group, an alkyl group, a heteroalkyl group, a haloalkyl group, an aryl group, an alkylaryl group, or an arylalkyl group.

In one embodiment of the present invention, R₁ and R₄ may each be independently a (C₁-C₂₀) alkyl group, a (C₆-C₂₀) aryl group, a (C₁-C₂₀) alkyl (C₆-C₂₀) aryl group, or a (C₆-C₂₀) aryl (C₁-C₂₀) alkyl group. However, in consideration of solubility of the phenazine-based compound, R₁ and R₄ may each be independently a methyl group, an ethyl group, or a propyl group.

In one embodiment of the present invention, R₂ and R₃ may each be independently a (C₁-C₅) alkylene group. As described above, when R₂ and R₃ are (C₁-C₅) alkylene groups, it is preferable because excellent solubility in a solvent and a high redox reaction rate can be attained.

The phenazine-based compound of the present invention may have high solubility in a non-aqueous solvent due to the effects of the substituents, and accordingly, a non-aqueous electrolyte for a redox flow battery having a high maximum energy density can be provided. In addition, since the phenazine-based compound is a multi-redox material capable of causing a two-stage reversible redox reaction when dissolved in a non-aqueous solvent, when the phenazine-based compound is used as an active material, a redox flow battery having a higher energy density can be provided. Specifically, the redox mechanism of the phenazine-based compound is shown in Reaction Scheme 1 below. However, the following Reaction Scheme 1 shows a chemical reaction according to one embodiment of the present invention, for example, a case where all of R₅, R₆, R₇, R₈, R₉, R₁₀, R₁₁, and R₁₂ are hydrogen.

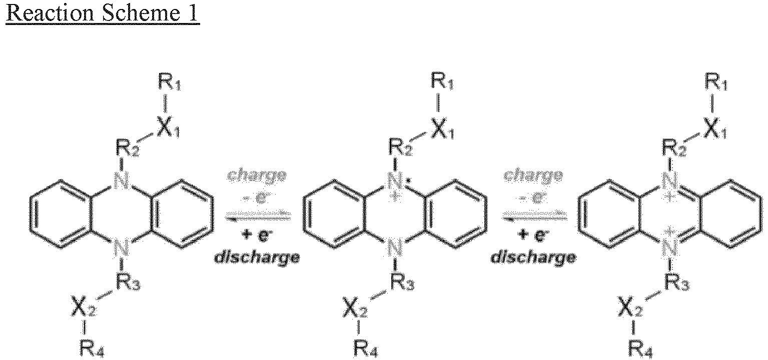

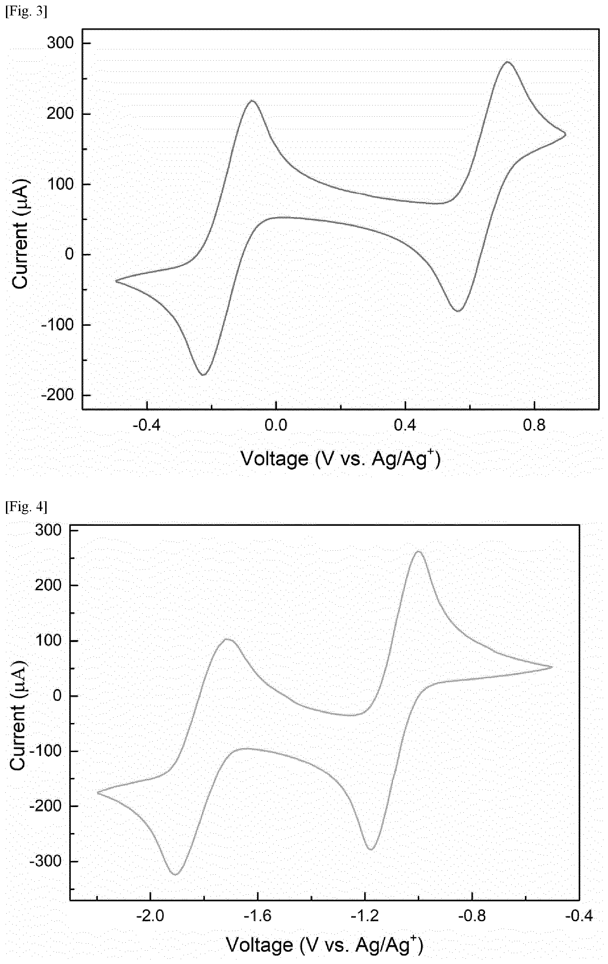

As shown in the above Reaction Scheme 1, in the phenazine-based compound of the present invention, two nitrogen moieties present in the molecular structure serve as redox motifs. Accordingly, two redox reactions sequentially occur, and as a result, two-stage reversible electrochemical activity is exhibited. In the case of an electrolyte prepared by dissolving such a compound in a solvent, when analyzed by cyclic voltammetry, two redox peaks may appear on a cyclic voltammogram during a redox reaction. Specifically, FIG. 3 shows a cyclic voltammogram of an electrolyte prepared using a phenazine-based compound according to one embodiment of the present invention.

In one embodiment of the present invention, the compound represented by Chemical Formula 1 may be BMEPZ (5,10-bis(2-methoxyethyl)-5,10-dihydrophenazine). Specifically, referring to a synthesis method of the BMEPZ, it can be seen that phenazine is first converted into 5,10-dihydrophenazine according to Reaction Scheme 2 below, and that BMEPZ is finally synthesized by a reaction between the 5,10-dihydrophenazine and 2-chloroethyl methyl ether. Since BMEPZ synthesized as thus has at least 10-fold higher solubility in a non-aqueous solvent than DMPZ conventionally used as an organic active material in a redox flow battery, a redox flow battery having high energy density can be provided.

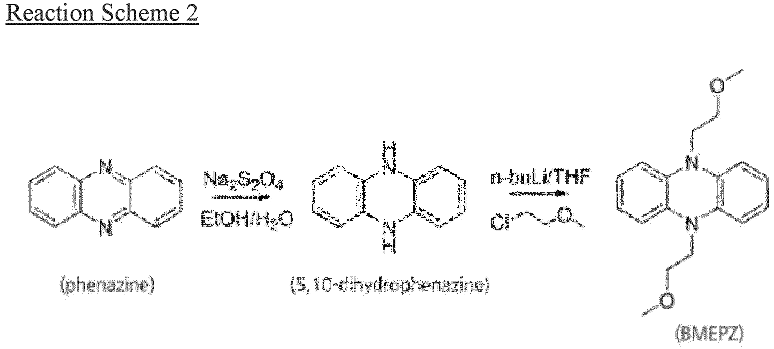

Another aspect of the present invention provides a redox flow battery electrolyte which includes a solute including the above-described phenazine-based compound; and a solvent.

In one embodiment of the present invention, the phenazine-based compound may be a cathode active material. Specifically, since the phenazine-based compound is an organic compound that undergoes a redox reaction in a relatively high potential region, it is preferable to apply the phenazine-based compound as a cathode active material, and when an organic compound that undergoes a redox reaction in a lower potential region than the phenazine-based compound is configured as an anode active material, an all-organic redox flow battery can be implemented.

In one embodiment of the present invention, when an electrolyte including the phenazine-based compound is scanned at a speed of 50 mVs⁻¹ or more in the range of -0.5 V to +1.0 V using cyclic voltammetry, two redox peaks may appear on a cyclic voltammogram. As described in the above Reaction Scheme 2, this phenomenon may occur due to two nitrogen moieties present in the molecular structure of the phenazine-based compound serving as redox motifs and thus causing two sequential redox reactions. Such a phenazine-based compound can provide a redox flow battery with a higher energy density as compared to organic materials only capable of producing one redox peak under the same conditions.

In one embodiment of the present invention, the solvent may have a phenazine-based compound solubility of 0.1 M or more and 10 M or less, specifically, 0.1 M or more and 5 M or less, or 0.1 M or more and 1.0 M or less. When the phenazine-based compound solubility is within the above range, it is preferable because an electrolyte having an appropriate viscosity and a redox flow battery having high energy density can be provided.

In one embodiment of the present invention, the solvent may include one or more solvents selected from the group consisting of acetonitrile, dimethyl carbonate, diethyl carbonate, dimethyl sulfoxide, dimethylformamide, propylene carbonate, ethylene carbonate, N-methyl-2-pyrrolidone, fluoroethylene carbonate, ethanol, and methanol. In addition, the solvent may be mixed with an aqueous solvent and used, and the aqueous solvent may include one or more selected from the group consisting of sulfuric acid, hydrochloric acid, and phosphoric acid.

Still another aspect of the present invention provides a redox flow battery which includes the above-described electrolyte. In this case, the redox flow battery may additionally include an anode active material. As the anode active material, any organic molecule commonly used in the art that is capable of a redox reaction may be used without limitation as long as it can undergo a redox reaction in a lower potential region than the phenazine-based compound.

In one embodiment of the present invention, the redox flow battery may additionally include an anode active material including an organic compound represented by the following Chemical Formula 2.

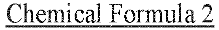

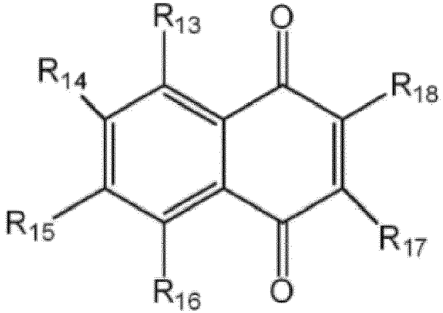

In Chemical Formula 2,
R₁₃, R₁₄, R₁₅, R₁₆, R₁₇, and R₁₈ are the same or different, and are each independently hydrogen, a halogen group, a hydroxyl group (-OH), a nitro group (-NO₂), a trifluoromethyl group (-CF₃), an alkoxy group, an alkyl group, a heteroalkyl group, a haloalkyl group, an aryl group, an alkylaryl group, or an arylalkyl group.

In one embodiment of the present invention, when an electrolyte including the anode active material is scanned at a speed of 50 mVs⁻¹ or more in the range of -2.0 V to -0.5 V using cyclic voltammetry, two redox peaks may appear on a cyclic voltammogram. That is, like the phenazine-based compound, the anode active material may also be a multi-redox material including two elements serving as redox motifs in the molecular structure. When such an anode active material having two redox peaks is used in combination with the phenazine-based compound (cathode active material) having two redox peaks to form an electrolyte, it is preferable because a redox flow battery having an optimal energy density can be provided.

In one embodiment of the present invention, the anode active material may include menadione. Since the menadione (MD) is a multi-redox organic material capable of causing a two-stage reversible redox reaction in a lower potential region than the phenazine-based compound, MD is preferably used as an anode active material.

The redox flow battery electrolyte of the present invention may additionally include a supporting electrolyte. The supporting electrolyte is added to additionally impart conductivity to the electrolyte, and any supporting electrolyte generally used in the art can be used without limitation. For example, the supporting electrolyte may be one or more selected from the group consisting of an alkyl ammonium salt, a lithium salt, and a sodium salt. In this case, the alkyl ammonium salt may be formed as a combination of an anion selected from among PF₆⁻, BF₄⁻, AsF₆⁻, ClO₄⁻, CF₃SO₃⁻, CF₃SO₃⁻, C(SO₂CF₃)₃⁻, N(CF₃SO₂)₂⁻, and CH(CF₃SO₂)₂⁻ and an ammonium cation which is a tetraalkylammonium cation having methyl, ethyl, butyl, or propyl as the alkyl. The lithium salt may be one or more selected from among LiPF₆, LiBF₄, LiAsF₆, LiClO₄, LiCF₃SO₃, LiCF₃SO₃, LiC(SO₂CF₃)₃, LiN(CF₃SO₂)₂ (simply referred to as "LiTFSI"), and LiCH(CF₃SO₂)₂ In addition, the sodium salt may be one or more selected from among NaPF₆, NaBF₄, NaAsF₆, NaClO₄, NaCF₃SO₃, NaCF₃SO₃, NaC(SO₂CF₃)₃, NaN(CF₃SO₂)₂, and NaCH(CF₃SO₂)₂. The supporting electrolyte can be used within a range generally used in the art, and for example, the supporting electrolyte can be used at a concentration of 0.1 M to 2 M in the electrolyte.

Meanwhile, the redox flow battery may be configured without limitation in a structure generally used in the art except that it should include the electrolyte of the present invention. Specifically, the redox flow battery may include a stack into which the electrolyte has been injected; a cathode, a separator, and an anode disposed in the stack; an electrolyte tank located outside the stack and storing the electrolyte; and a pump used to circulate the electrolyte between the tank and the stack.

Hereinafter, the present invention will be described in detail by way of exemplary embodiments. However, the exemplary embodiments of the present invention may be implemented in various modified forms, and the scope of the present invention is not to be construed as being limited to the exemplary embodiments described below. The exemplary embodiments of the present specification are provided to more fully describe the present invention to those of ordinary skill in the art.

### Example 1: Synthesis of BMEPZ

2 g of phenazine and 20 g of sodium hydrosulfite (Na₂S₂O₄) were dissolved in a mixture of 50 mL of ethanol and 200 mL of distilled water, stirred at 75 °C for 10 minutes, and then purified, and thus 1.8 g of 5,10-dihydrophenazine was obtained.

Subsequently, the 1.8 g of 5,10-dihydrophenazine and 11.4 mL of 2-chloroethyl methyl ether were dissolved in a mixture of 11 mL of an n-butyllithium solution (2.5 M in hexane) and 25 mL of tetrahydrofuran (THF), stirred at 25 °C for 12 hours, and then purified, and thus 2.07 g of BMEPZ was obtained.

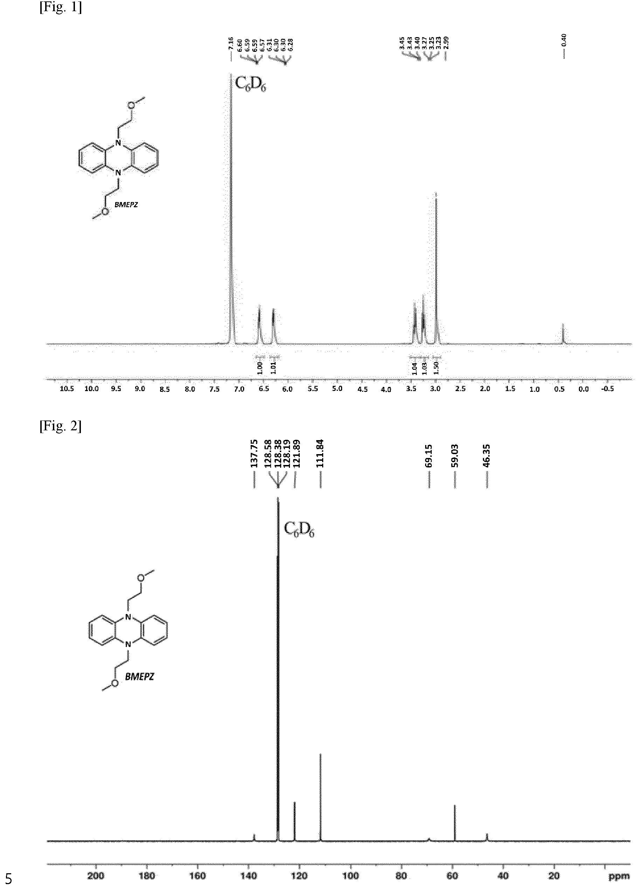

The ¹H-NMR and ¹³C-NMR spectra of the above BMEPZ were obtained using Bruker Avance-300 and Avance-500 NMR spectrometers, respectively, and the measured values are shown below, and the ¹H-NMR and ¹³C-NMR spectra are shown in FIGS. 1 and 2, respectively. The chemical formula of BMEPZ obtained as thus is as follows.

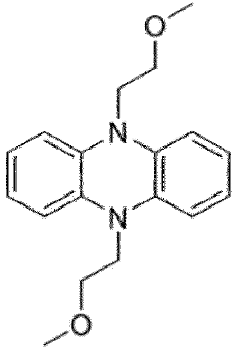

¹H-NMR (300 MHz, C6D6) δ(ppm): 6.60-6.57(m, 4H), 6.31-6.28(m, 4H), 3.43(t, J = 6.3 Hz, 4H), 3.25(t, J = 6.3 Hz, 4H), 2.99(s, 6H).

¹³C-NMR (125 MHz, C6D6) δ(ppm): 137.75, 111.84, 69.15, 59.03, 46.35.

### Example 2: Preparation of electrolyte including BMEPZ

A cathode electrolyte was prepared by dissolving the BMEPZ obtained in Example 1 in a solvent at a concentration of 0.01 M. In this case, as the solvent, an acetonitrile solvent including tetrabutylammonium hexafluorophosphate (TBAPF₆) at a concentration of 0.1 M was used.

### Example 3: Preparation of electrolyte including combination of BMEPZ and MD

An anode electrolyte was prepared by dissolving MD (Sigma-Aldrich) in a solvent at 0.01 M. In this case, as the solvent, an acetonitrile solution including TBAPF₆ at a concentration of 0.1 M was used.

Subsequently, a combined electrolyte was prepared by mixing the above anode electrolyte and the cathode electrolyte prepared in Example 2 in a volume ratio of 1:1.

### Comparative Example 1: Provision of DMPZ

Commercially available DMPZ (Tokyo Chemical Industry Co., Ltd.) was purchased and provided. The chemical formula of the DMPZ is as follows.

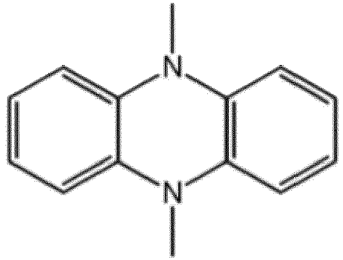

### <Evaluation methods>

### 1. Solubility

Each of the phenazine-based compounds provided according to Example 1 and Comparative Example 1 was dissolved in an acetonitrile solution including TBAPF₆ at a concentration of 0.1 M, and the maximum soluble amounts of the phenazine-based compounds were identified, and the results are shown in the following Table 1.

**[Table 1]**

| | Solubility (M) |
|---|---|
| Example 1 | 0.5 |
| Comparative Example 1 | 0.05 |

Referring to Table 1, it can be seen that the phenazine-based compound produced in Example 1 had a significantly higher solubility than the phenazine-based compound of Comparative Example 1.

### 2. Evaluation of electrochemical properties

Cyclic voltammetry was used to analyze the electrochemical properties of the cathode electrolyte, anode electrolyte, and combined electrolyte prepared in Examples 2 and 3.

The testing was conducted at a scanning speed of 300 mVs⁻¹ using each of the electrolytes. The cells used for measuring cyclic cyclic voltammograms were configured using an Ag/Ag⁺ electrode formed by dissolving AgNO₃ in an acetonitrile solvent at a concentration of 0.3 M as a reference electrode, carbon felt as a working electrode, and platinum as a counter electrode.

FIGS. 3 to 5 show the cyclic voltammograms of the cathode electrolyte prepared in Example 2 and the anode electrolyte and the combined electrolyte prepared in Example 3, respectively.

Referring to FIG. 3, it can be seen that two redox peaks appear in a -0.5 V to +1.0 V potential region of the cyclic voltammogram, and from this, it can be confirmed that the phenazine-based compound is applicable as an active material for a redox flow battery in which two electrons react.

Referring to FIG. 4, it can be seen that two redox peaks appear in a -2.0 V to -0.5 V potential region of the cyclic voltammogram, and from this, it can be confirmed that the MD is applicable as an active material for a redox flow battery in which two electrons react.

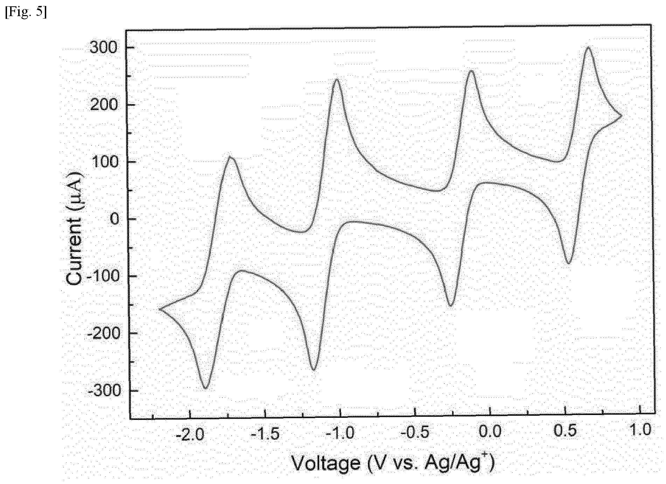

In addition, referring to FIG. 5, the two redox peaks appearing on the right side of the cyclic voltammogram are peaks corresponding to BMEPZ, and the two redox peaks appearing on the left side are redox peaks corresponding to MD Therefore, it can be seen that BMEPZ, which undergoes a redox reaction in a higher potential region, is applicable as a cathode active material, and MD, which undergoes a redox reaction in a lower potential region, is applicable as an anode active material.

While the present invention has been described above through exemplary embodiments and drawings, it should be understood that the present invention is not limited thereto, and that various changes and modifications can be made by those of ordinary skill in the art to which the present invention pertains within the technical spirit of the present invention and the scope of the appended claims and equivalents thereof.

## Claims

1. A phenazine-based compound represented by the following Chemical Formula 1: 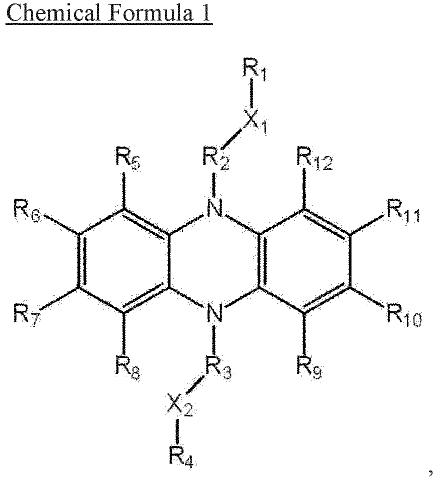 wherein, in Chemical Formula 1:
X₁ and X₂ are the same or different, and are each independently oxygen (O), sulfur (S), or silicon (Si);
R₁ and R₄ are the same or different, and are each independently an alkyl group unsubstituted or substituted with an acetal group or an ether group; an aryl group unsubstituted or substituted with an acetal group or an ether group; an alkylaryl group unsubstituted or substituted with an acetal group or an ether group; or an arylalkyl group unsubstituted or substituted with an acetal group or an ether group;
R₂ and R₃ are the same or different, and are each independently an alkylene group unsubstituted or substituted with an acetal group or an ether group; and
R₅, R₆, R₇, R₈, R₉, R₁₀, R₁₁, and R₁₂ are the same or different, and are each independently hydrogen, a halogen group, a hydroxyl group (-OH), a nitro group (-NO₂), a trifluoromethyl group (-CF₃), an alkoxy group, an alkyl group, a heteroalkyl group, a haloalkyl group, an aryl group, an alkylaryl group, or an arylalkyl group.

2. The phenazine-based compound of claim 1, wherein the R₁ and the R₄ are each independently a (C₁-C₂₀) alkyl group, a (C₆-C₂₀) aryl group, a (C₁-C₂₀) alkyl (C₆-C₂₀) aryl group, or a (C₆-C₂₀) aryl (C₁-C₂₀) alkyl group.

3. The phenazine-based compound of claim 1, wherein the R₁ and the R₄ are each independently a methyl group, an ethyl group, or a propyl group.

4. The phenazine-based compound of claim 1, wherein the R₂ and the R₃ are each independently a (C₁-C₅) alkylene group.

5. The phenazine-based compound of claim 1, wherein the compound represented by Chemical Formula 1 is 5,10-bis(2-methoxyethyl)-5,10-dihydrophenazine (BMEPZ).

6. A redox flow battery electrolyte comprising:
a solute including the phenazine-based compound of claim 1; and
a solvent.

7. The redox flow battery electrolyte of claim 6, wherein the phenazine-based compound is a cathode active material.

8. The redox flow battery electrolyte of claim 6, wherein, when the electrolyte including the phenazine-based compound is scanned at a speed of 50 mVs⁻¹ or more in a range of -0.5 V to +1.0 V using cyclic voltammetry, two redox peaks appear on a cyclic voltammogram.

9. The redox flow battery electrolyte of claim 6, wherein a solubility of the phenazine-based compound in the solvent is 0.1 M or more and 10 M or less.

10. The redox flow battery electrolyte of claim 9, wherein the solvent includes one or more solvents selected from the group consisting of acetonitrile, dimethyl carbonate, diethyl carbonate, dimethyl sulfoxide, dimethylformamide, propylene carbonate, ethylene carbonate, N-methyl-2-pyrrolidone, fluoroethylene carbonate, ethanol, and methanol.

11. A redox flow battery comprising the electrolyte of any one of claims 6 to 10.

12. The redox flow battery of claim 11, further comprising an anode active material including an organic compound represented by the following Chemical Formula 2: 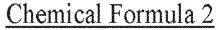 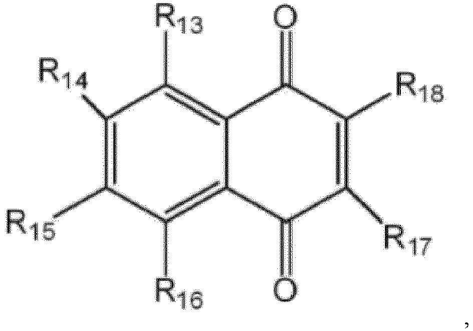 wherein, in Chemical Formula 2, R₁₃, R₁₄, R₁₅, R₁₆, R₁₇, and R₁₈ are the same or different, and are each independently hydrogen, a halogen group, a hydroxyl group (-OH), a nitro group (-NO₂), a trifluoromethyl group (-CF₃), an alkoxy group, an alkyl group, a heteroalkyl group, a haloalkyl group, an aryl group, an alkylaryl group, or an arylalkyl group.

13. The redox flow battery of claim 12, wherein the anode active material includes menadione (MD).

14. The redox flow battery of claim 12, wherein, when the electrolyte including the anode active material is scanned at a speed of 50 mVs⁻¹ or more in a range of -2.0 V to -0.5 V using cyclic voltammetry, two redox peaks appear on a cyclic voltammogram.
